Europäisches Patentamt

European Patent Office

Office européen des brevets

(11) Publication number: **0 295 749**

**A1**

## (12) EUROPEAN PATENT APPLICATION

(21) Application number: **88201200.8**

(51) Int. Cl.⁴: **A61K 39/39**

(22) Date of filing: **10.06.88**

(30) Priority: **15.06.87 NL 8701384**

(43) Date of publication of application:
**21.12.88 Bulletin 88/51**

(84) Designated Contracting States:
**AT BE CH DE ES FR GB GR IT LI LU NL SE**

(71) Applicant: **DUPHAR INTERNATIONAL RESEARCH B.V**
**C.J. van Houtenlaan 36**
**NL-1381 CP Weesp(NL)**

(72) Inventor: **Masihi, Kemuel N.**
**c/o Octrooibureau Zoan B.V. P.O. Box 140**
**NL-1380 AC Weesp(NL)**
Inventor: **Hilgers, Lucas A. Th.**
**c/o Octrooibureau Zoan B.V. P.O. Box 140**
**NL-1380 AC Weesp(NL)**

(74) Representative: **Muis, Maarten et al**
**OCTROOIBUREAU ZOAN B.V. P.O. Box 140**
**NL-1380 AC Weesp(NL)**

(54) **Sulpholipopolysaccharides as stimulators of the non-specific defence mechanism.**

(57) It has been found that sulpholipopolysaccharides (SLP's), which are known to enhance the immunogenic activity of certain antigens (i.e. stimulate the specific immune system), can be used to built up a non-specific immunity against pathogenic micro-organisms.

EP 0 295 749 A1

## Sulpholipopolysaccharides as stimulators of the non-specific defence mechanism.

The invention relates to the use of sulpholipopolysaccharides (SLP's) as stimulators of the non-specific defence mechanism or immune system.

Immunisation of man and animal against infection diseases of old has been based on the principle of an immune response of the body to the administration of an antigen. By the administration of an antigen the immune system is triggered to form antibodies against the pathogenic antigen. It is sometimes possible to stimulate the immune response by administering an adjuvant in addition to an antigen.

In this manner a resistance can be built up against a given antigen, that is to say a specific immunity against a specific disease.

It is known (Immunology, 60, (1987), pp. 141-146) that SLP's can stimulate specific immune reactions, i.e. they are suitable as adjuvants for enhancing the immunogenic activity of certain antigens.

It is also known that under certain circumstances (for example, in the case of stress) the resistance to diseases is influenced. In such circumstances, micro-organisms which normally only have a sub-clinic effect, may cause severe diseases or may even be fatal.

It has now been found that a non-specific immunity of the body against pathogenic micro-organisms can be built up by administering SLP's. As a result of the administration of SLP's components of the non-specific branch of the immune system of the body are stimulated, as a result of which an increased resistance to viruses, bacteria and other (micro)organisms is formed. Furthermore, an increased resistance to tumours and the like could be produced in this manner.

The non-specific immune response of SLP's built up from a synthetic polysaccharide to which lipid groups and sulphate groups are coupled covalently, can be established and measured by means of an animal model.

In this test-animal model the SLP's to be tested are administered to mice. The SLP's are preferably administered in the form of an oil-in-water emulsion suitable for injection purposes, the SLP's being dissolved in the oil phase or in the water phase. Squalane may, for example, be used as the oil phase. A suitable emulsion is obtained by emulsifying 1% of squalane with the SLP's dissolved therein in a physiological saline solution or in water.

One week to 10 days after administration of the emulsion the mice are challenged by the administration of a quantity of a pathogenic organism, for example, a dose of $100 \times LD_{50}$ of a pathogenic influenza virus. The increased resistance of animals which have previously been treated with the SLP emulsion appears from a lower death rate as compared with control animals which have been pre-treated with an emulsion without SLP or which have not been pre-treated at all, and from a longer average life of killed animals in the test group compared with animals from the control groups.

Polysaccharides which are suitable for application according to the invention are, for example, copolymers of sucrose and epichlorohydrin, for example, the copolymer which is commercially available under the name of Ficoll.

Suitable lipid groups which may be used in the SLP's according to the invention are, for example, lauroyl, myristoyl, palmitoyl and stearoyl. The SLP's according to the invention comprise 0.01-0.8, preferably 0.01-0.2 lipid groups, and 0.1-1.6, preferably 0.2-1.6 sulphate groups per unit of monosaccharide. Good results are obtained with SLP's which comprise a small number of lipid groups and a large number of sulphate groups, for example, 0.01 lipid groups and 0.6 sulphate groups per unit of monosaccharide.

The invention will now be described in greater detail with reference to the ensuing specific example.

EXAMPLE

A small number of lipid groups (palmitoyl groups) and a large number of sulphate gorups were coupled in known manner to a copolymer of sucrose and epichlorohydrin (the polysaccharide Ficoll having a molecular weigh of 400kD of Pharmacia, Uppsala, Sweden). The purified conjugate was dissolved in an oil-in-water emulsion suitable for injection purposes.

Groups of 10-20 mice were injected intravenously with 500 µg of an SLP dissolved in the oil phase of a 1% squalane emulsion in water. The emulsion without SLP or nothing was administered to animals in the control group(s). Seven days later all animals were infected with an aerosol which comprises an influenza virus (A/PR/8/34 H1N1) pathogenic to mice ($100 \times LD_{50}$ per mouse).

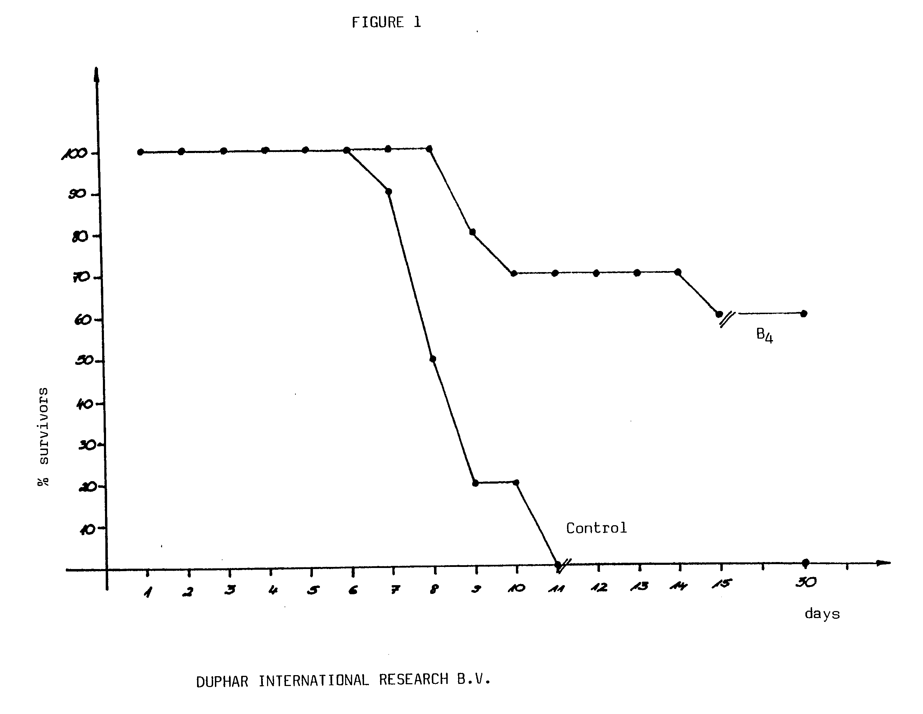

It appears from the table below that the average life of the dead animals of the test group is longer than that of the control groups (A1 (only polysaccharide) and control),i.e. 10.4 and 8.2 days, respectively, after infection. It further appears from Figure 1 that the death rate in mice which have previously been treated with the SLP-emulsion is much lower ($\leq 50\%$) than in the untreated control group (death rate 100%). It will hence be obvious that administration of SLP's activates non-specific components of the immune system and thus increases the resistance to an infectious agent.

TABEL

| Effect of synthetic SLP's on the survival time of mice after infection with influenza virus ($100 \times LD_{50}$ as an aerosol) | | | | |
|---|---|---|---|---|
| SLP | Palmitoyl* | Sulphate* | Average survival time in days | |
| | | | test 1 | test 2 |
| A1 | 0 | 0 | $8.2 \pm 0.7$ | - |
| A3 | 0 | 0.2 | $8.6 \pm 1.1$ | - |
| B2 | 0.01 | <0.1 | $9.2 \pm 1.2$ | $9.6 \pm 1.6$ |
| B3 | 0.01 | 0.2 | $10.5 \pm 1.3$ | $10.4 \pm 2.1$ |
| B4 | 0.01 | 0.6 | $10.4 \pm 1.3$ | $10.8 \pm 2.9$ |
| Control | - | - | $8.5 \pm 0.6$ | $8.2 \pm 0.9$ |

* number of palmitoyl groups and sulphate groups, respectively per unit of monosaccharide

**Claims**

1. A method of stimulating the non-specific immune system of the human or animal body, characterized in that sulpolipopolysaccharides are used.

2. A method as claimed in Claim 1, characterized in that a sulpholipopolysaccharide dissolved in an oil-in-water emulsion to be injected is used.

3. A method as claimed in Claim 2, characterized in that a sulpholipopolysaccharide dissolved in an oil-in-water emulsion of 1% of squalane in a physiological saline solution is used.

4. A method as claimed in Claim 1, characterized in that a copolymer of sucrose and epichlorohydrin to which lipid groups and sulphate groups are coupled covalently is used as a sulpholipopolysaccharide.

5. A method as claimed in Claim 4, characterized in that Ficoll having a molecular weight of approximately 400kD is used as a sulpholipopolysaccharide.

6. A method as claimed in Claim 5, characterized in that a sulpholipopolysaccharide is used which comprises 0.01-0.8 lipid groups and 0.1-1.6 sulphate groups per unit of monomer.

7. A method as claimed in Claim 5 or 6, characterized in that a sulpholipopolysaccharide is used which comprises palmitoyl groups as lipid groups.

8. An injection composition which comprises sulpholipopolysaccharide and is suitable for use with the method as claimed in Claim 1.

DIR 0395

FIGURE 1

B4

Control

days

% survivors

DUPHAR INTERNATIONAL RESEARCH B.V.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,P | IMMUNOLOGY, vol. 60, 1987, pages 141-146; L.A.T. HILGERS et al.: "Synthetic sulpholipopolysaccharides: novel adjuvants for humoral immune responses" * Whole article * | 1-8 | A 61 K 39/39 |
| A | US-A-4 590 181 (McCARTHY) * Column 2, line 50 - column 6, line 27 * | 1-8 | |
| A | US-A-4 057 685 (F.C. McINTIRE) | | |
| A | FR-A-2 484 258 (MARUYAMA CHISATO) | | |

TECHNICAL FIELDS
SEARCHED (Int. Cl.4)

A 61 K

The present search report has been drawn up for all claims

| Place of search | Date of completion of the search | Examiner |
|---|---|---|
| THE HAGUE | 16-09-1988 | REMPP G.L.E. |

EPO FORM 1503 03.82 (P0401)